(19) Europäisches Patentamt
European Patent Office
Office européen des brevets

(11) Publication number: **0 529 247 A2**

# (12) EUROPEAN PATENT APPLICATION

(21) Application number: **92111441.9**

(51) Int. Cl.[5]: **G01N 21/64**, C09K 11/06

(22) Date of filing: **06.07.92**

(30) Priority: **27.08.91 US 750781**

(43) Date of publication of application:
**03.03.93 Bulletin 93/09**

(84) Designated Contracting States:
**DE FR GB IT NL SE**

(71) Applicant: **BOC HEALTH CARE, Inc.**
**575 Mountain Avenue**
**Murray Hill, New Jersey 07974(US)**

(72) Inventor: **Milo, Charles S.**
**30652 Rainbow View Drive**
**Agoura Hills, California 91301(US)**

(74) Representative: **Dipl.-Phys.Dr. Manitz**
**Dipl.-Ing.Dipl.-Wirtsch.-Ing. Finsterwald**
**Dipl.-Phys. Rotermund Dipl.-Chem.Dr. Heyn**
**B.Sc.(Phys.) Morgan**
**Robert-Koch-Strasse 1**
**W-8000 München 22 (DE)**

## (54) Optical chemical sensors and sensing materials.

(57) A sensor for monitoring a chemical condition such as pH includes a luminescent material insensitive to the condition to be sensed. The luminescent material can absorb light in two excitation wavelength bands and emit response light as a result of absorption in either band. The sensor also includes a dye having absorptivity in one such excitation wavelength band which varies with the condition to be monitored. The luminescent material is illuminated with excitation light in the two excitation wavelength bands alternately. Response to the one excitation varies with the condition, whereas response to the second does not vary and provides an internal reference.

The invention also provides vinyl functionalized photoactive materials such as dyes and luminescent materials which can be incorporated into vinyl polymers. The vinyl functionalized photoactive material maybe made by reacting a vinyl benzyl halide reagent with a photoactive material having a phenol group. The vinyl benzyl functionality is bound to the phenol group in the ortho position relative to the OH group of the phenol.

EP 0 529 247 A2

## BACKGROUND OF THE INVENTION

The present invention relates to measurement of chemical and physical phenomena by using photoactive materials such as dyes and luminescent materials sensitive to the condition to be monitored, to instruments, components and polymeric materials useful in such measurements, and to methods and monomers useful in making such polymers.

Chemical conditions such as the pH of a solution can be monitored by observing the changes in optical properties of substances which vary in their optical properties according to the particular chemical condition. Perhaps the simplest examples of such a system are the dyes commonly referred to as indicators. Such indicators change in color depending upon the pH of the surrounding environment, so that the pH can be measured by observing this change with the naked eye.

*Grattan et al.,* "Dual Wavelength Optical Fiber Sensor for pH Measurement," *Biosensors* 3 (1987-88) 17-25, describes a variant of this approach in which two light sources are used alternately to illuminate a chemical solution containing a phenol red pH indicator. The phenol red indicator has light absorptivity in the green wavelength band which varies in accordance with the pH of the solution, whereas the light absorptivity of the phenol red in the red wavelength band does not vary appreciably with pH. The apparatus includes two light sources, one for emitting red light and another for emitting green light. These are actuated alternately, and the light reflected back from the solution is monitored by a photocell. The output of the photocell is sampled by two separate sample-and-hold devices, one while the red light is present and another while the green light is present. The ratio of the two signals obtained provides a measure of the solution pH.

*Peterson et al.,* Fiber Optic pH Probe for Physiological Use, *Anal. Chem.* 1980, Vol. 52, pp. 864-869, discloses a probe incorporating a dye indicator bound to microspheres in the tip of a fiber optic probe, with a "cycling filter wheel" to provide alternate illumination in green and red bands with monitoring of the intensity of the reflected light during each phase of illumination and computation of a ratio between these two signals.

Other instruments utilize luminescent materials, i.e., fluorescent or phosphorescent materials which emit response upon exposure to excitation light. Ordinarily, the emitted light is of a longer wavelength than the excitation light. In one approach exemplified by German Democratic Republic Patent Specification No. 106,086, luminescent material sensitive to the phenomenon to be monitored is provided on the end of a fiber optic element. The intensity of emission is monitored by directing excitation light through the fiber optic and monitoring the response light returned via the fiber optic. The particular luminescent material is sensitive to the condition to be monitored in that the intensity of the response light varies with the condition. For example, many common luminescent materials are subject to "quenching" or diminution of luminescent intensity upon exposure to oxygen. These materials may be employed to measure oxygen concentrations. To compensate for changes in the response light caused by deterioration of the luminescent material and/or the optical instrument, a reference luminescent material may be provided which is either insensitive to the condition to be monitored or else is shielded from that condition.

*Scheggi et al.,* "pH Sensing by Fiber Optics," *Optica Acta* 1986, Vol. 33, No. 12, pp. 1587-1597, offers a general survey of optical techniques for sensing pH, including a phenol red-based sensor using green light which is absorbed to varying degrees depending upon pH, and red light which is not absorbed. The same reference discloses, as an alternative, a fluorescence-based sensor using hydroxypyrene trisulfonic acid ("HOPSA") in a fiber optic.

In the variant of the fluorescence approach, changes in the absorption spectrum of the fluorescent dye are exploited to provide a measure of the condition to be monitored. Thus, *Gehrich, et al.,* Optical Fluorescence and Its Application to an Intravascular Blood Gas Monitoring System, IEEE. Transactions on Bio-Medical Engineering, Vol. BME 33, No. 2, pp. 117-132 (1986), discloses an instrument in which a pH-sensitive fluorescent dye (hydroxypyrene trisulfonic acid) is mounted on a fiber optic. This particular fluorescent dye absorbs light at 460 nm wavelength and at 410 nm wavelength, and emits at 520 nm. The absorption changes with pH. At relatively basic conditions, absorption at 460 nm is favored and absorption at 410 nm is disfavored, whereas the reverse is true under acidic conditions. The dye is alternately illuminated with 460 nm and 410 nm excitation light, and the resulting luminescent response light is monitored. Under basic conditions, where the dye tends to absorb more at 460 nm, the emission in response to the 460 nm illumination will be greater than that produced in response to 410 nm illumination. Under acidic conditions, the reverse will be true. Therefore, the ratio of the response light intensity upon 410 nm illumination to response light intensity upon 460 nm illumination provides a measure of the pH.

*Zhujun et al.,* "A Fluorescent Sensor for Quantifying pH in the Range from 6.5 to 8.5," *Analytica Chemica Acta,* Vol. 160 (1984), pp. 47-55, describes a similar fluorescent sensor incorporating HOPSA as

a fluorescent material with excitation at two different wavelengths and calculation of the ratio between the fluorescence intensity resulting from excitation at one wavelength relative to the fluorescence intensity resulting from excitation at another wavelength.

My own co-pending, commonly assigned United States Patent Application Serial No. 07/480,995, filed February 16, 1990, discloses sensing apparatus incorporating two luminescent materials. One of these luminescent materials may be substantially insensitive to the condition to be monitored, whereas the other may be sensitive to such condition so that its luminescent response varies with the condition to be monitored. The two luminescent materials may be sensitive to excitation light in different bands, so that excitation light in one band will excite the first luminescent material, whereas excitation light in the other will excite the second luminescent material. Excitation light in these two bands may be applied in alternating sequence and the response light emitted by the two luminescent materials may be sampled in synchronism with such excitation to provide signals representing the two luminescent responses. These may be compared to one another. Use of the two luminescent materials provides an internal reference or calibration signal. Because the output of the instrument is based upon the comparison between the two signals, phenomena which cause degradation of both luminescent materials, such as photo-bleaching, will not materially affect the measurement.

Fluorescence based sensors offer some significant advantages, particularly for bio-medical applications such as monitoring chemical conditions prevailing within a living organism. It is ordinarily desirable to construct sensors for such measurements using relatively small fiber optics and small masses of active sensing substances at the tips of the fiber optics. Sensors based only upon absorbent dyes typically depend upon light passed through the fiber optic and reflected or back-scattered at the tip of the fiber optic. With practical illumination intensities, the light reflected back from the tip may be extremely dim and difficult to measure. To secure adequate reflected light, rather complex or bulky mechanical arrangements may be required. The fluorescence-based instruments can alleviate these drawbacks.

However, fluorescence-based sensors have certain drawbacks of their own. In particular, the available fluorescent materials may be insensitive to the particular condition to be monitored, or else may be sensitive to a broad range of chemical species and hence susceptible to interfering substances. By comparison, many different colormetric dyes sensitive to particular chemical conditions are available. In particular, there are numerous pH-sensitive light absorbing dyes. Many of these are substantially insensitive to chemical conditions other than pH, at least under normal conditions of use.

*Jordan et al.,* "Physiological pH Fiber-Optic Chemical Sensor Based on Energy Transfer," *Anal. Chem.*, 1987, Vol. 59, pp. 437-439; *Walt et al..,* "Preparation of Small Diameter Sensors for Continuous Clinical Monitoring", SPIE, Vol. 713, optical Fibers in Medicine, II (1986), pp. 144-149; and *Walt,* U.S. Patent No. 4,822,746, all propose sensors in which light absorbing dyes are used in conjunction with luminescent materials. The luminescent and light absorbing materials may be disposed in a polymeric mass at the end of a fiber optic. Excitation light directed into the fiber optic causes the luminescent material to emit response light. A part of the emitted response light is absorbed by the light absorbing material. As the light absorbing material is sensitive to the condition to be monitored, the degree of such absorption, and hence the net amount of response light transmitted back through the fiber optic, varies with the condition to be monitored.

This approach suffers from drawbacks of its own in that it does not provide an internal reference standard. The dye and/or the luminescent material may degrade during use of the instrument, as by "photo-bleaching" or degradation caused by the excitation light, which may either increase or decrease the intensity of response light transmitted back through the fiber optic. It may be difficult to distinguish a change caused by such degradation from a change caused by variation in the conditions to be monitored. Thus, despite the substantial scientific effort devoted towards development of optically based sensors heretofore, there remain substantial needs for still further improvements in these devices.

Significant efforts have also been made towards development of better methods and materials for incorporating photo active materials such as dyes and luminescent materials into the structures of sensors. It is often necessary to place the photoactive material in a solid matrix so that the photoactive material can be exposed to the condition to be monitored by placing the solid matrix in contact with the environment. For example, in physiological sensors for monitoring chemical conditions such as pH, $PCO_2$ or $PO_2$ of the blood in a living organism, a dye and/or a luminescent material may be disposed in a solid matrix and the solid matrix may be mounted at the end of the fiber optic so that the matrix can be exposed to the blood by inserting the fiber optic into a blood vessel. The solid matrix normally must be permeable to at least some substances in the surroundings so that such substances can contact the photoactive material disposed within the matrix.

Where the photoactive material is simply mechanically incorporated within the matrix, the photoactive material may be leached from the matrix during use of the instrument. Accordingly, there have been efforts heretofore to copolymerize the dye with other monomers so that the dye is integrally incorporated as part of the polymeric matrix. Thus, *Peterson,* U.S. Patent No. 4,194,877, notes that certain pH indicating dyes, such as phenol red, brilliant yellow and rosolic acid can be copolymerized with acrylic monomers, notably acrylamide, when the monomer is polymerised in the normal fashion. The same reference mentions the possibility of copolymerization with methylmethacrylate or hydroxymethylmethacrylate, but offers no specific directions as to how to conduct a process with these monomers. The process of this reference apparently is limited to operation with particular dyes which are inherently susceptible to copolymerization, and apparently requires the presence of an acrylic monomer.

*Kelsius, Inc.,* Published Patent Application WO 88/0553, describes processes for making copolymers incorporating photoactive substances such as dyes and luminescent materials by first functionalizing the photoactive material with a compound incorporating a vinyl moiety and then polymerizing the resulting product, typically with another monomer also incorporating a vinyl moiety. The synthesis procedure used in the Kelsius publication requires that the photoactive material have functional groups specifically reactive with the vinyl-bearing compound. Thus, Example 14 of this publication shows the procedure starting with cresol red, a pH-sensitive dye. The dye is first treated with N-bromosuccinimide to brominate the methyl group of the cresol red. The product is then reacted with allylamine so that the amine group of the allylamine directly replaces the bromine. This provides a functionalized cresol red with allylamine moiety bonded to the methyl group of the cresol red in the location occupied by the bromine. This functionalized cresol red is then copolymerized with acrylamide. Example 7 of the same publication shows treatment of fluorescein amine with acryloyl chloride so that the acryloyl moiety directly displaces one hydrogen on the amine group of the fluorescein amine, leaving the acryloyl moiety bonded to the amine group. This product is then copolymerized with acrylamide. Munkholm *et al.,* "Polymer Modification of Fiber Optic Chemical Sensors as a Method of Enhancing Fluorescence Signal for pH Measurement," *Anal. Chem.*, 1986, Vol. 58, pp. 1427-1430, uses a similar reaction between fluorescein amine and acryloyl chloride to form the vinyl-bearing acryloyl fluorescein monomer for copolymerization. Here again, the vinyl-bearing moiety is directly substituted on an existing functional group of the photoactive material.

A very significant drawback of the reaction scheme taught by the Kelsius and Munkholm references is that the photoactive material must incorporate a suitable functional group for reaction with the vinyl-bearing moiety. This substantially limits the choice of photoactive materials which can be processed and may also require preliminary steps, such as the bromination referred to in the case of cresol red, to prepare a material having a suitable functional group. Further, the photoactive materials often incorporate hydroxyl groups bonded to aromatic rings, which hydroxyl groups play a part in the photoactivity of the substance. In the scheme proposed by the Kelsius published application, the vinyl-bearing reagent and the conditions under which it is reacted with the photoactive material must be selected so that the vinyl reagent compound does not substantially react with the hydroxyl group These factors limit the utility of the Kelsius process and the types of products which can be formed.

*Iyer et al.*, U.S. Patent 4,925,268 discloses a method of covalently linking phenol red to a pre-existing polymer such as a methylmethacrylate and methacrylamido propyltrimethlammonium copolymer by first reacting an aminoarylalkylamine with the polymer and then reacting that product with phenol red. See column 6 of the *Iyer et al.* disclosure.

*Zhujun et al.,* "Polyvinyl Alcohol as a Substrate for Indicator Immobilization for Fiber-Optic Chemical Sensors," *Analytical Chem,* Vol. 61, pp. 202-205 (1988) suggests a scheme for immobilizing photoactive materials in which cyanuric chloride is reacted with polyvinyl alcohol so as to link the cyanuric chloride with the polyvinyl alcohol and then the linked cyanuric chloride is reacted with fluorescein amine so as to link the fluorescein amine to the cyanuric chloride and hence the polyvinyl alcohol. Here, again, the reaction scheme requires that the photoactive material incorporate a functional group reactive with the cyanuric chloride. This substantially limits the materials which can be linked to polymers in this process. Thus, despite the substantial efforts devoted heretofore to development of polymers incorporating photoactive materials and methods of preparing the same, there still remain substantial needs for improvements in these areas as well.

## SUMMARY OF THE INVENTION

The present invention addresses the needs noted above.

One aspect of the present invention provides an apparatus for monitoring a condition. The apparatus includes a support and a luminescent material mounted on the support. The luminescent material is

adapted to emit light, referred to herein as "response light," within a band of wavelengths referred to herein as the "response band." The luminescent material is arranged to emit this response light in response to measurement excitation light, within a band of wavelengths referred to as the "measurement band of excitation wavelengths." The luminescent material is also adapted to emit the response light in response to so-called "reference excitation light" within a band of wavelengths referred to herein as the "reference band of excitation wavelengths" which is different from the measurement band. Thus, either measurement excitation light or reference excitation will cause the luminescent material to emit the response light. The luminescent material itself need not be sensitive to the condition to be monitored, and desirably is substantially insensitive to that condition.

The apparatus also includes a light absorbing material sensitive to the condition to be monitored. The light absorbing material desirably is mounted on the support so that the light absorbing material can be exposed to the condition to be monitored. The light absorbing material may be a colorimetric dye, and has absorptivity for light in the measurement band of excitation wavelengths which varies in response to the condition to be monitored. The absorptivity of the light-absorbing material in the reference band preferably does not vary significantly in response to the condition to be monitored. The apparatus also includes illumination means for providing measurement and reference excitation light in a time-varying scheme such that the ratio of the intensity of light in the measurement band to the intensity of light in the reference band varies with time, and transmitting the reference and measurement excitation light to the luminescent material so that at least the transmitted measurement excitation light passes through the light absorbing material. Most preferably, the excitation means is arranged to provide the measurement and reference excitation light alternately, so that no reference excitation light is provided while the measurement excitation light is being provided, and so that no measurement excitation light is provided while the reference excitation light is provided.

The apparatus further includes sensor means for monitoring response light emitted by the luminescent material. The sensor means desirably includes signal processing means for providing a measurement signal indicating the magnitude of response light emitted in response to the measurement excitation light and for providing a reference signal indicating the magnitude of the response light produced responsive to the excitation light. For example, where the measurement and reference excitation light are provided alternately, the luminescent material will emit response light responsive to the measurement excitation light during illumination by the measurement excitation light and will emit response light in response to the reference excitation light during illumination by the reference excitation light. The signal processing means may include means such as a photodetector for providing a response signal representing the magnitude of the response light and means for sampling the response signal in synchronism with the alternating reference and excitation light.

Because the absorptivity of the light absorbing material in the measurement band varies with the condition to be monitored, the amount of measurement excitation light reaching the luminescent material will vary with the condition to be monitored. Thus, the intensity of the response light emitted in response to the measurement excitation light will also vary with the condition to be monitored, and the measurement signal representing the magnitude of this response light will also vary. The response light produced in response to the reference excitation signal, and the reference signal indicating the magnitude of that light provides an internal reference in the instrument itself. Degradation or partial loss of the luminescent material will change both the measurement signal and the reference signal, and hence will not substantially alter the output signal.

Because there is no requirement for the luminescent material to be sensitive to the particular condition to be monitored, a wide range of luminescent materials can be employed. The light absorbing material desirably incorporates a dye sensitive to the condition to be monitored. Substantially any dye which undergoes a change in light absorptivity in a band corresponding to an absorption band of the luminescent material can be employed. As a wide range of dyes sensitive to various chemical and physical conditions are known, the instrument can be adapted readily to monitoring many different conditions. In particular, the light absorbing material may incorporate a pH-sensitive dye such as phenol red or phenolphthalein for monitoring the pH of the environment. The instrument can be adapted to sense $CO_2$ concentration in the environment by incorporating a $CO_2$ sensitive buffer adapted to change its pH depending upon $CO_2$ concentration in the matrix of the pH-sensitive dye.

The support may include a fiber optic having proximal and distal ends. The light absorbing and luminescent materials may be mounted adjacent the distal end of the fiber optic. In a particularly preferred arrangement, the light absorbing material and the luminescent material are incorporated in the same solid matrix such as a mass of a polymer bonded to the tip of the fiber optic. In this arrangement, both the measurement excitation light and the reference excitation light will pass through at least part of the light

absorbing material to the luminescent material, and the response light will likewise pass through the light absorbing material. Preferably, the light absorbing material has substantially zero absorptivity in the response wavelength band or else has absorptivity in that band which does not vary with the condition to be monitored.

A further aspect of the present invention provides a condition-sensitive element for incorporation in apparatus as aforesaid. This element desirably includes the support, luminescent material and light absorbing material as described above with reference to the instrument, and may also include means for connecting the support to a source of reference and measurement excitation light. Most desirably, this sensitive element incorporates a fiber optic as a support, the luminescent and light absorbing materials being mounted at a distal end of the fiber optic, and the connecting means being adapted to connect the proximal end of the fiber optic to the source of illumination.

Further aspects of the present invention provide methods of making photoactive polymers, vinyl functionalized photoactive materials useful in manufacture of such polymers, and methods of making the vinyl functionalized photoactive materials. The vinyl functionalized photoactive materials according to this aspect of the present invention typically include a photoactive moiety, such as a dye or a luminescent substance, having one or more unsubstituted or partially substituted phenol moieties and having one or more vinyl benzyl moieties bound to the phenol moieties at the ortho position with respect to the -OH group of each such phenol moiety. Stated another way, the vinyl functionalized photoactive materials according to this aspect of the present invention preferably conform to the general structural formula:

$$R_1[R_2]_{n_1} \qquad \text{I}$$

wherein:

$n_1$ is an integer greater than or equal to 1;

$R_1$ is a photoactive moiety having one or more phenol moieties, the number of said phenol moieties being at least equal to $n_1$;

each $R_2$ is independently selected from the group consisting of moieties according to the formula:

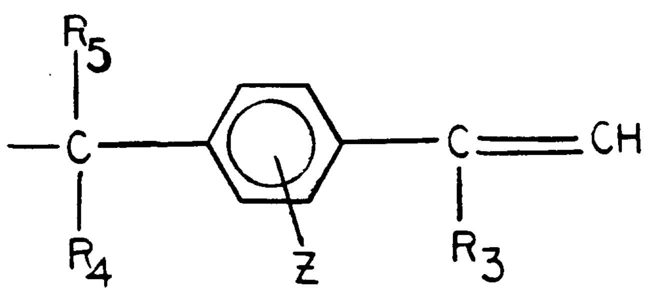

II

each of $R_3$, $R_4$ and $R_5$ being independently selected from the group consisting of aryl, lower alkyl and H; and

Z represents the moieties on the aromatic ring, selected from the same group as $R_3$-$R_5$,

each said $R_2$ being bound to a separate one of said phenol moieties of $R_1$ at the ortho position with respect to the -OH group of such phenol moiety.

Preferably, the photoactive moiety is a pH-sensitive dye moiety. Thus, the vinyl functionalized photoactive material most preferably is a pH-sensitive photoactive material. Particularly preferred pH-sensitive photoactive materials have the general structural formula:

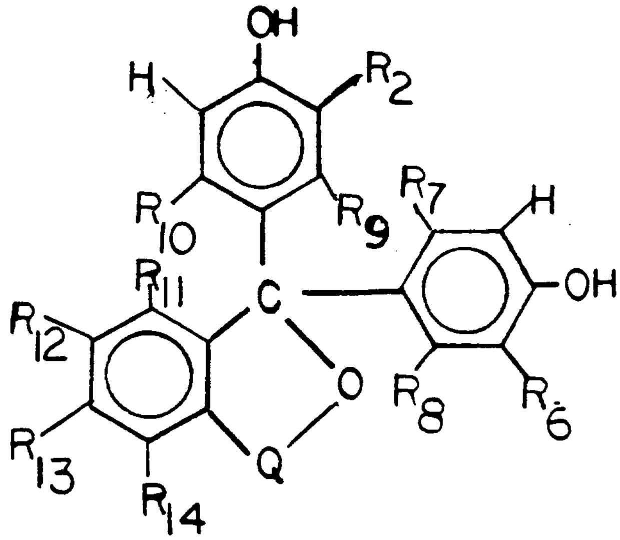

III

in which Q is a moiety selected from the group consisting of

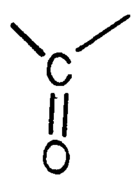

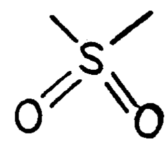

IV & V

and $R_2$ is as aforesaid. Each of $R_7$ through $R_{14}$ desirably is independently selected from the group consisting of lower alkyls, H and the halogens. $R_6$ may be selected from the group consisting of lower alkyls, H, the halogens and $R_2$. That is, $R_6$ as well as $R_2$ may be a vinyl benzyl group, in which case the dye has two vinyl functionalities. The preferred structures thus include mono- and di-vinylfunctionalized phenol red (where Q is sulfone) and mono- and divinyl functionalized phenolphthalein (where Q is carbonyl).

In the preferred methods of making the vinyl functionalized photoactive materials, a photoactive compound having one or more phenol moieties is reacted with a vinyl benzyl halide reagent according to the formula X-$R_2$ in which $R_2$ is the substituted or unsubstituted vinyl benzyl moiety described above, and X is a halogen selected from the group consisting of F, Cl and Br. In this reaction, $R_2$ replaces the hydrogen of the hydroxyl group in the phenol moiety of the photoactive compound to form the ether of the vinyl benzyl group and the photoactive material on an intermediate. Replacement of the hydrogen in the hydroxyl group of a phenol is commonly referred to as a Williamson reaction.

In the next step of the reaction, the ether intermediate is reacted under conditions which promote a rearrangement in which the $R_2$ moiety displaces a proton at the ortho position relative to the ether bond, and the proton, in turn, replaces the $R_2$ moiety of the ether, so as to reform the hydroxyl group in the same position as originally occupied by that group, and leave the $R_2$ vinyl benzyl moiety bonded to the phenol group at the ortho position with respect to that hydroxyl group. The step of reacting the intermediate to provide this rearrangement may occur simultaneously with the step of forming the intermediate or in a separate stage of the reaction after the intermediate has been formed. The conditions required to promote such rearrangement typically include only reasonable application of heat.

As previously noted, many desirable photoactive compounds incorporate phenol moieties and these are believed necessary to the photoactivity of the materials. The synthesis method according to this aspect of the present invention uses these hydroxyl groups as reactive sites during synthesis, but then reforms them. Accordingly, substantially any photoactive material having phenol groups with one or, preferably, both positions ortho to the -OH group unsubstituted can be employed as a starting material. There is no need to prepare derivatized versions of the photoactive material, and no need to conduct the reaction under special conditions to protect the hydroxyl groups.

The most preferred photoactive materials for use as starting materials in the reaction are those directly analogous to the preferred vinyl functionalized materials as discussed above. Thus, the preferred starting

materials include pH-sensitive dyes, and particularly pH-sensitive dyes according to the formula:

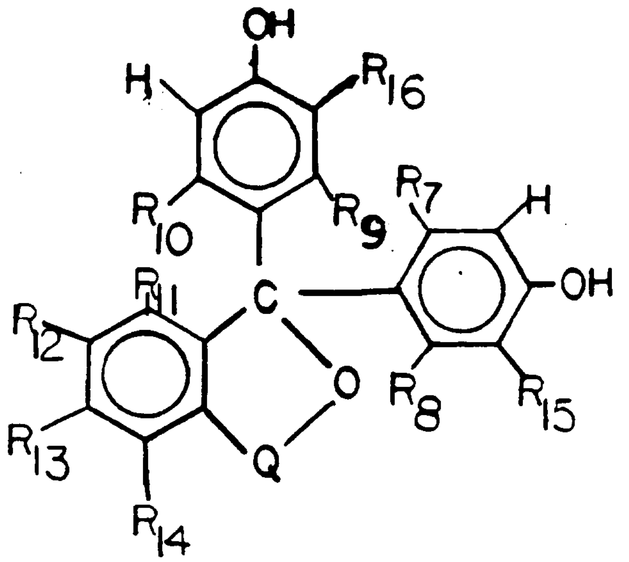

VII

in which Q is carbonyl or sulfone, $R_7$ through $R_{16}$ are independently selected from the group consisting of lower alkyls, H and halogens. Preferably, at least one of $R_{15}$ and $R_{16}$ is H. Phenol red and phenolphthalein and particularly preferred.

The method of making a polymeric photoactive material includes the step of polymerizing a vinyl functionalized photoactive material as discussed above. Desirably, the polymerizing step involves copolymerizing the vinyl functionalized photoactive material with one or more other, different vinyl monomers. The polymerization reaction may be conducted under conventional vinyl polymerization conditions, using catalysts or initiators commonly used in polymerization of vinyl monomers.

The resulting polymer has first repeating units according to the formula:

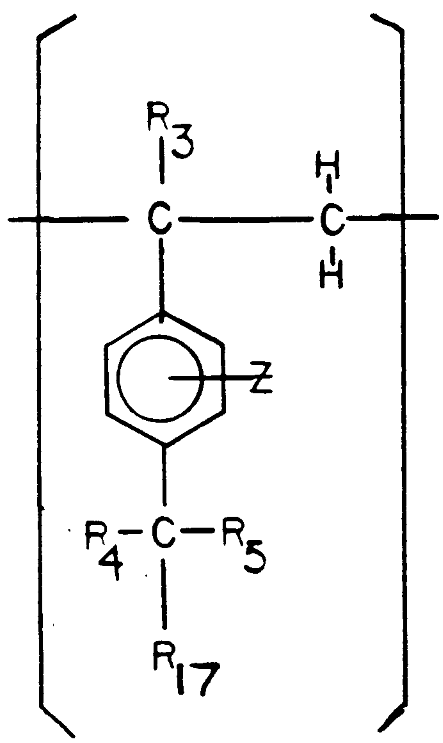

VIII

in which $R_3$, $R_4$, $R_5$ and Z are as aforesaid, and $R_{17}$ represents the photoactive moiety having one or more phenol moieties, the $R_4$-C-$R_5$ moiety being bound to one of the phenol moieties of $R_{17}$ at the ortho position with respect to the -OH group of that phenol moiety.

Where the polymer is a copolymer, it will include additional uniform or non-uniform additional repeating units according to the formula:

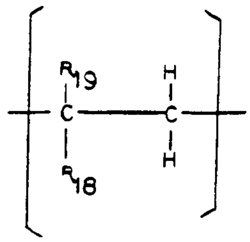

IX

in which $R_{18}$ and $R_{19}$ are independently selected from the group consisting of H and organic moieties. Preferably, $R_{18}$ and $R_{19}$ are selected from the group consisting of H, alkyl, substituted and unsubstituted benzyl and esters. Particularly preferred comonomers for incorporation in a copolymer with the photoactive monomer are methylacrylate, methylmethacrylate and monomers incorporating an ionic moiety such as an ammonium moiety. As will be appreciated, a wide variety of vinyl monomers are available, and hence the polymer may be provided with a wide variety of physical and chemical characteristics. In particular, the preferred ionic and acrylate comonomers render the polymer permeable to water, and hence promote intimate contact between the photoactive moieties and $H^+$ in aqueous surroundings, so that the polymer can serve as a pH indicator. Trimethylammonium methylmethacrylate methosulfate is a particularly preferred comonomer.

As discussed above, the vinyl functionalized photoactive moiety may incorporate two vinyl functionalities attached to phenol moieties in the photoactive material. In this case, the first repeating units of the polymer would be substantially in accordance with the formula:

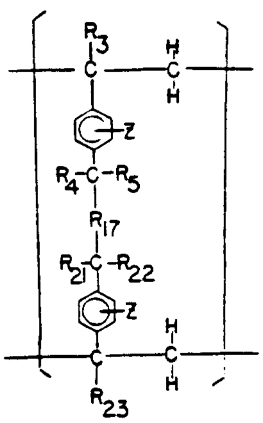

X

in which $R_{21}$, $R_{22}$ and $R_{23}$ are independently selected from the group consisting of aryl, lower alkyl and H. Most desirably, a polymer incorporating difunctional repeating units of this form also incorporates additional repeating units defining generally linear vinyl polymeric chains. The $-CR_3-CH_2$ moiety and the $-CR_{23}-CH_2$ moiety of each of these difunctional first repeating units desirably are polymerized in separate ones of these linear chains. That is, the chains desirably are cross-linked through the photoactive $R_{17}$ moieties.

These and other objects, features and advantages of the present invention will be more readily apparent from the detailed description of the preferred embodiments set forth below, taken in conjunction with the accompanying drawings.

## BRIEF DESCRIPTION OF THE DRAWINGS

FIG. 1 is a schematic, partially block diagrammatic view of apparatus in accordance with one embodiment of the present invention.

FIG. 2 is a fragmentary, diagrammatic view on an enlarged scale of certain components used in the apparatus of FIG. 1.

FIG. 3 is a graph depicting certain properties of photoactive materials employed in the apparatus of FIG. 1.

FIG. 4 is a timing diagram illustrating operation of certain components in the apparatus of FIG. 1.

FIG. 5 is a fragmentary view depicting portions of apparatus according to a further embodiment of the invention.

## DETAILED DESCRIPTION OF THE PREFERRED EMBODIMENT

Apparatus according to one embodiment of the invention includes a sensing element 10 incorporating an elongated fiber optic 12 having a proximal end 14 and a distal end 16. The proximal end 14 is provided with a coupler 18 for releasably attaching the probe to the remainder of the apparatus as discussed below. A mass 20 of a light-transmitting polymer permeable to $H^+$ ions is bonded to the distal end of 16 of fiber optic 12. Mass 20 incorporates both a light absorbing dye and a luminescent material. Each of these photoactive materials may be present in the mass as a dispersion of the photoactive material in the polymer of the mass, such as particles of the photoactive material physically disposed in the mass, or as a solution of the photoactive material in the polymer. Alternatively, either or both of these photoactive materials may be present as part of the polymer itself, i.e., as photoactive moieties within the polymer molecules themselves.

The spectral characteristics of the dye and the luminescent material are schematically depicted in FIG. 3. The luminescent material is adapted to absorb light over a relatively broad range of wavelengths as indicated by curve 22. In that curve, the height of the curve at any point indicates the relative absorptivity at the particular wavelength denoted by position along the horizontal axis marked "Wavelength." Thus, the luminescent material will absorb light in a first or measurement band of excitation wavelengths 23, as well as in a second or reference band of excitation wavelengths 25, and will emit light within a response band of wavelengths 27 in response to the absorbed light. Curve 24 shows the relative intensity of the response light emitted by the luminescent material at various wavelengths. As seen in FIG. 3, the maximum emission occurs at a longer wavelength than the maximum absorption, although the emission and absorption spectrum may overlap, as indicated by the overlapping of curves 22 and 24. The absorption and emission spectra 22 and 24 of the luminescent material do not vary appreciably with pH.

The absorption spectrum of the pH-sensitive light absorbing dye at one pH is shown by curve 26, whereas the absorption spectrum of the same dye at another, lower pH is shown by curve 28. As will be apparent from FIG. 3, the dye has absorptivity for light within measurement wavelength band 23 which varies substantially with pH. The dye has very little absorptivity for light in the reference band of wavelengths 25, and such absorptivity as it may have in that band does not vary substantially with pH. As is also apparent from FIG. 3, the dye does not have substantial absorptivity for response light emitted by the luminescent material within response wavelength band 27.

The apparatus further includes a first or measurement light source 30 (FIG. 1) including a light emitting diode 32, a diode driver 34 adapted to actuate diode 32 to emit light and an optical filter 36 adapted to pass light from diode 32 only within the first or measurement band of wavelengths 23 (FIG. 3). The apparatus further includes a second or reference light source 38 including a light emitting diode 40, a diode driver 42 and an optical filter 44 which is arranged to pass only that portion of the light from diode 40 lying within the second or reference wavelength band 25.

An optical coupler 46 is connected to receive light passing through filters 36 and 44. The optical coupler has an appropriate fitting 48 for mechanically engaging the fitting 18 on the proximal end of the fiber optic. Optical coupler 46 also includes internal optical components 50 schematically for receiving and directing light. The optical coupler is arranged so that when the fiber optic is retained in position on the coupler by inter-engaged fittings 18 and 48, the proximal end 14 of the fiber optic is in light transmitting relationship with the internal components 50 of the optical coupler. Thus the light directed through the optical couplers can be sent into the proximal end of the fiber optic, and light passing through the fiber optic and out from the distal end of the fiber optic will be directed into the optical components of the coupler. The particular mechanical arrangement schematically depicted in FIG. 2 is merely representative of the numerous possible mechanical and optical arrangements which may be employed for detachably securing the proximal end 14 of the fiber optic 12 in operative, optically interactive relationship with the internal elements of optical coupler 46. Devices and methods for detachably securing fiber optics to other optical elements are known in the art, and any such known devices can be used. Coupler 46 is arranged to receive light passing through filters 36 and 44 and to direct that light into the proximal end of the fiber optic.

The apparatus further includes an optical filter 52 arranged to pass only light falling within the response wavelength band 27 (FIG. 3) and a photodetector 54 adapted to produce an electrical signal directly related to the intensity of light falling thereon. Optical coupler 46 is arranged to direct light passing from the proximal end of the fiber optic through filter 52 onto photodetector 54. The electrical output of photodetector 54 is connected to the input of an amplifier 56. The output of amplifier 56 is connected to input terminals of switches 58 and 60. The output terminal of switch 58 is connected to the input of an integrator 62, whereas the output of switch 60 is connected to the input of a separate integrator 64. The outputs of integrators 62 and 64 are connected to an electronic dividing circuit 66 arranged to divide the magnitude of the signal from integrator 62 by the magnitude of the signal from integrator 64 and to provide an output signal representing the result of that division. A meter 68 is connected to the output of divider 66 so that the output signal will be shown on the meter. A timing and control unit 70 is connected to control diode drivers 34 and 42 and switches 58 and 60.

In one method according to the invention, the distal end 16 of fiber optic 12, and hence polymer mass 20, are disposed in an environment to be monitored, such as in the interior of a blood vessel V of a living subject by threading the fiber optic into the blood vessel through a conventional intravenous catheter 72. As polymer mass 20 is in contact with the blood inside vessel V, and as polymer mass 20 is substantially permeable to $H^+$ ions, the pH within mass 20 rapidly reaches equilibrium with the pH of the blood. Accordingly, the photoactive materials within the mass are exposed to the pH prevailing within the blood.

Control unit 70 actuates the diode drivers 34 and 42 alternately, and hence actuates measurement light source 30 and reference light source 38 alternately. Curve 74 (FIG. 4) schematically depicts the light output from source 30 versus time. As source 30 produces measurement excitation light within the first or measurement excitation wavelength band 23, curve 74 illustrates the variation in the amount of measurement excitation light directed into fiber optic 12 versus time. The measurement excitation light is applied only during measurement illumination intervals 76, and not at other times. Similarly, curve 78 depicts the magnitude of the reference excitation light in band 25 supplied by reference light source 38, and indicates that the reference light is applied only during reference illumination intervals 80, interspersed between the measurement illumination intervals 76. The measurement excitation light and reference excitation light pass through the fiber optic and into mass 20.

As the light absorbing dye and the luminescent material are substantially equally dispersed throughout mass 20, the excitation light passing from the distal end of the fiber optic to the luminescent material passes through the light absorbing dye before reaching the luminescent material. The amount of dye intervening between the distal end of the fiber optic and any particular portion of the luminescent material varies with the position of that particular portion of luminescent material within mass 20. Thus, those parts of the luminescent material immediately adjacent the distal end 16 of the fiber optic receive excitation light which has passed through only a small amount of light absorbing dye, whereas those portions of the luminescent material at the ultimate tip of mass 20, remote from the fiber optic, receive excitation light which has passed through substantially all of the dye. The net effect, however, is that the luminescent material on average receives excitation light after such excitation light has passed through a substantial amount of the light absorbing dye.

The intensity of response light emitted from the luminescent material in mass 20 is indicated by curve 82. During each measurement illumination interval 76 (FIG. 4), while the luminescent material is being illuminated by the measurement excitation light, it begins to emit response light including response light in response with length band 27. This emission rises rapidly to a steady state value during illumination, and decays promptly after the illumination ceases. Desirably, the delay between the end of each measurement illumination interval 76 and the next succeeding reference illumination interval 80 is such that the response light emission has substantially completely decayed before commencement of the next response illumination interval. Thus, the luminescent material emits light at a substantially steady intensity in response to the measurement excitation light during measurement response intervals 84 substantially synchronized with measurement illumination intervals 76. Likewise, the luminescent material emits light in response to the reference illumination light during reference emission intervals 86, substantially synchronized with reference illumination intervals 83.

As the measurement excitation light is absorbed to a substantial degree by the dye in passing to the luminescent material, the amount of measurement excitation light reaching the luminescent material is less than the amount of reference excitation light reaching the luminescent material. Because the amount of response light emitted by the luminescent material is directly related to the amount of excitation light applied, the magnitude of the response light during measurement response intervals 84 is less than the magnitude of the response light during reference response intervals 86. This difference in response is directly related to the degree of absorption by the dye in measurement excitation band 23, i.e., to whether

the dye is strongly absorbing as indicated by curve 26 (FIG. 3) or weakly absorbing as indicated by curve 28. As discussed above, the dye will shift from curve 26 to curve 28 depending upon the pH of the environment.

The response light is routed through fiber optic 12, optical coupler 46 and filter 52 to photodetector 54. Photodetector 54 provides an electrical signal which is amplified by amplifier 56. The magnitude of the signal from photodetector 54, and hence the magnitude of the signal from amplifier 56 is directly related to the intensity of the response light. Inasmuch as the light absorbing dye in mass 20 does not have appreciable absorptivity for light in response band 27, and whatever absorptivity it may have does not vary substantially, the relationship between the electrical response signal from amplifier 56 and the magnitude of the emissions by the luminescent material in mass 20 is substantially constant. Thus, the response signal from amplifier 56 will vary in substantially the same way as the response emissions indicated by curve 82 (FIG. 4). The electrical response signal will have a greater magnitude during reference response intervals 86 than during measurement response intervals 84.

Timing and control unit 70 actuates switches 58 and 60 to sample the response signal from amplifier 56 in an alternating sequence synchronized with the alternating sequence of measurement and reference illumination intervals and hence synchronized with the alternating sequence of measurement and reference response intervals. Thus, switch 58 is turned on so as to route a sample of the response signal to integrator 62 during each measurement response interval 84, whereas switch 60 is turned on to route a sample of the response signal to integrator 64 during each reference response interval 86. Integrator 62 therefore accumulates an electrical measurement signal 62 directly related to the magnitude of the measurement response light, whereas integrator 64 accumulates an electrical reference signal indicating the magnitude of the reference response light. Dividing circuit 66 divides the measurement signal from integrator 62 by the reference signal from integrator 64, and produces an output signal representing the resulting quotient.

The measurement response signal supplied to divider 66 will represent the measurement response light. As the pH at the mass 20 increases, the absorption spectrum of the dye in mass 20 shifts from spectrum 28 (FIG. 3) towards spectrum 26, and hence its absorptivity in measurement excitation band 23 increases. This increases the attenuation of measurement excitation light passing from fiber optic 12 to the luminescent material in mass 20, and hence decreases the amount of measurement excitation light reaching the luminescent material. This causes a decrease in the measurement response light and a corresponding decrease in the measurement signal supplied by integrator 62. By contrast, the reference signal provided by integrator 64 will be substantially unaffected by the pH prevailing at mass 20. Thus, as pH increases the measurement signal decreases whereas the reference signal remains constant, so that the output signal from divider 66 decreases with increasing pH.

Loss or degradation of the luminescent material will affect both the measurement and the reference signals, and hence will not materially change the output signal from divider 66. Likewise, changes in the optical system such as changes in the transmission characteristics of fiber optic 12 will affect both measurement and reference signals and hence will not materially change the output signal. Thus, the reference signal provides an internal compensation feature which allows the instrument to provide reliable results. This compensation scheme is particularly useful where the probe or condition-sensitive element (the fiber optic and mass) may be replaced often. Thus, the fiber optic and mass may be a disposable unit. The instrument will still give useful results even where factors such as the optical transmission properties of the fiber optic or the fluorescent properties of the material used in various disposable units are not identical with one another.

The physical configurations discussed above can be varied in many ways. Thus, it is not essential for the dye or light absorbing material to be incorporated in a common mass with the luminescent material. As illustrated in FIG. 5, the dye or light absorbing material may be disposed in one mass 120 immediately adjacent the distal end 116 of the fiber optic, whereas the luminescent material may be disposed in another polymer mass 121 attached to the distal end of mass 120, remote from the fiber optic. Thus, the dye is disposed in a separate mass, interposed between the distal end of the fiber optic and the luminescent material. In this arrangement, the polymer of the dye containing mass 120 desirably is permeable to substances in the environment to be monitored, whereas mass 121 may be formed from an impermeable polymer or else coated with a separate coating (not shown) to render it impermeable to substances in the environment which might influence the response characteristics of the luminescent material. Thus, a luminescent material which might be inherently sensitive to the condition to be monitored, or to other interfering conditions, will maintain substantially constant properties. The condition-sensitive element need not include a fiber optic as the support for the photoactive materials. Thus, the support may be a container adapted to contain a sample of a fluid or a conduit adapted to permit flow of a fluid. The photoactive materials may be mounted on the interior of such a container or conduit. It is most desirable, but not always

essential, to incorporate the photoactive materials in polymeric masses. Thus, the photoactive materials can be present in one or more liquid solutions disposed in suitable chambers on the support, or encapsulated in microcapsules on the support. Alternatively, the photoactive materials can be coated on substrates such as polymer or glass beads secured to the support. These approaches, however are less preferred.

The apparatus can be used to monitor conditions other than the pH of the surroundings. Thus, colorimetric dyes sensitive to conditions other than pH can be used instead of pH-sensitive dyes. By way of example, the colorimetric dyes referred to in Table I, below can be employed with oxazine 1 perchlorate as the luminescent material for monitoring concentration of the analytes indicated in the table. Literature references further describing each such dye are also shown in the table.

TABLE I

| Analyte | Colorimetric Dye | Literature Reference |
|---|---|---|
| $NH_3$ | ETH 5350 | Analytical Chem., Vol. 63, pp. 640-644 (1991) |
| $Fe^{3+}$ | N,N-dimethyl-p-phenylenediamine with $H_2O_2$ | Analytical Chem., Vol. 62, pp. 738-742 (1990) |
| $Ca^{2+}$ | ETH 5294 | Analytical Chem., Vol. 60, pp. 2573-2577 (1988) |
| Amino Acids | Diketo-hydrin dilidene-diketolhydrin diamine | SPIE Vol. 990, Chemical Biochemical and Environmental Applications of Fibers pp. 70-77 (1988) |

The pH-sensing apparatus can be used to monitor $CO_2$ concentration where the mass of polymer containing the pH sensitive dye is impregnated with a bicarbonate buffer solution or otherwise incorporates bicarbonate or carbonate functionality. Desirably, a $CO_2$-permeable, water-impermeable membrane or coating such as a silicone or polyvinylchloride is provided on the polymer mass to retain the buffer. For $CO_2$ sensing and for pH sensing in the physiologic pH range (about 7 to about 7.8), the most preferred dyes are phenol red and phenolphthalein. Apparatus using a pH-sensitive colorimetric dye can also be used to monitor other conditions. For example, apparatus using phenol red as the colorimetric dye can be used to monitor relative humidity or temperature where the phenol red is chemically isolated from the surrounding environment. Also, the apparatus using pH-sensitive colorimetric materials can be used to monitor other analytes where the pH-sensitive material is supplemented with additional reagents as indicated in Table II.

TABLE II

| Analyte | Additional Reagent |
|---|---|
| $NH^3$ | Ammonium buffer solution |
| Urea | Urease |
| Glucose | Glucose Oxidase |
| Lactate | Lactate Mono-Oxidase |
| Penicillin | Penicillinase |

The luminescent materials which can be employed include oxazine 1 perchlorate, oxazine 4 perchlorate, oxazine 170 perchlorate, Nile Blue A Perchlorate, Cresyl Violet Perchlorate, Sulforhodamine 101, Sulforhodamine $B_1$, rhodamine, porphyrin, Texas Red and lissamine and derivatives thereof.

In the apparatus discussed above, the dye has substantially constant, low absorptivity in the reference band. However, the instrument can operate in similar fashion even where the absorptivity of the dye in the reference band varies with the condition to be monitored. The degree or direction of such variation should be different from the variation of the absorptivity in the measurement band, so that the ratio of measurement-band absorptivity to reference-band absorptivity changes with the condition to be monitored.

As discussed above, it is desirable to incorporate the photoactive materials into the polymeric masses as photomoieties chemically bound in the polymer itself. In preferred preparation methods according to the invention, this is accomplished by preparing a vinyl functionalized photoactive material and then polymerizing that vinyl functionalized photoactive material with other vinyl monomers. The starting material for

the vinyl functional step of the process desirably include a photoactive material having one or more phenol moieties with at least one and desirably both positions ortho to the OH group of such phenol moiety unsubstituted, i.e., occupied by H. Apart from the OH groups of the phenol moieties, the photoactive starting material desirably is free of groups susceptible to reaction with the vinylbenzyl reagent, such as hydroxyl, carboxylic acid, amine and ester moieties. pH-sensitive light absorbing dyes having the aforementioned phenol moieties constitute one particularly useful class of photoactive materials. Particularly preferred pH sensitive photoactive materials are those according to the general structural formula:

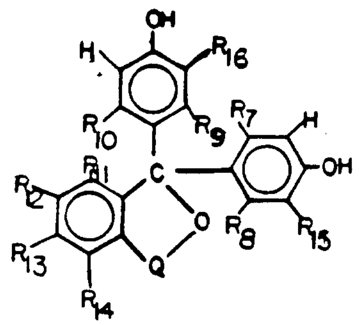

VII

in which: Q is selected from the group consisting of:

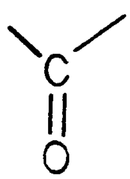

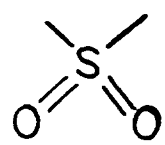

IV & V

and R7 through R16 are independently selected from the group consisting of lower alkyls, H and halogens. Preferably, at least one of R15 and R16 is H. The class of compounds where Q is the sulfone moiety includes phenol red and derivatives thereof, whereas the class of compounds where Q is carbonyl includes phenolphthalein and derivatives thereof.

Among the other photoactive materials which can be employed in the polymerization process are luminescent materials such as fluorescein and its derivatives, and 7-hydroxycoumarin and its derivatives.

The vinylbenzyl halide reagent desirably is according to the structural formula:

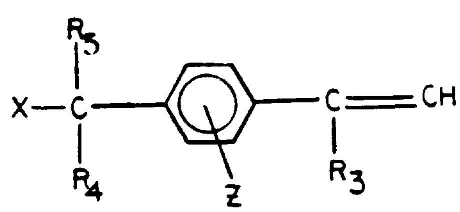

VI

in which each of R3, R4 and R5 is independently selected from the group consisting of substituted and unsubstituted aryl, substituted and unsubstituted lower alkyl, halogens and H, and in which X is a halogen selected from the group consisting of F, Cl and Br. The vinyl benzyl halide reagent can additionally have one or more substituents on the aromatic ring. In this formula, Z represents the moieties on the aromatic ring, which can be selected from the same moieties as R3-R5. Particularly preferred vinyl benzyl halide reagents have H as R4 and R5 and desirably have no non-hydrodgen substituents on the aromatic ring, i.e., all Z moieties are H. H is the preferred moiety at the $R_3$ position as well. The most referred vinyl benzyl halide reagent is para vinyl benzyl chloride.

The vinyl benzyl halide reagent and the photoactive material may be reacted by mixing these two reagents in a dry solvent such as dimethyl formamide or DMSO, preferably under alkaline conditions to form a reaction mixture. Upon such contacting, the starting materials react to form a vinyl benzyl ether of the photoactive material, with the vinyl benzyl moieties linked via ether links to the photoactive moiety at the

sites formerly occupied by the hydroxyl groups of the photoactive moiety. Desirably, at least about 1 mole of vinyl benzyl halide reagent is provided per mole of photoactive material. Where the photoactive material incorporates plural phenol groups, the molar ratio of vinyl benzyl halide reagent to photoactive material should be at least equal to the number of such phenol groups in photoactive material molecule. Thus, where the photoactive material includes 2 phenol groups, the molar ratio of vinyl benzyl halide to photoactive material should be at least about 2:1.

In the next stage of the process, the vinyl benzyl ether intermediate is held under conditions which promote rearrangement. Typically, application of an elevated temperature such as between about 100°C and about 250°C, and desirably about 130°C-170°C for a period in excess of about 4 hours, preferably in excess of about 24 hours and desirably between about 48 and 72 hours is sufficient to promote such rearrangement. The ether intermediate may be subjected to rearrangement conditions in the same reaction mixture as used for the first reaction step, and the rearrangement conditions may be applied before the first reaction step is completed so that the first reaction proceeds simultaneously with the rearrangement. Thus, in the simplest form of the process, the photoactive material and vinyl benzyl halide reagent are mixed with the solvent and to form a reaction mixture, and that reaction mixture is subjected to the elevated temperature conditions discussed above. Alternatively, the ether intermediate can be isolated and then dissolved in another dry solvent to form a second reaction mixture, and that second reaction mixture may be subjected to the elevated temperature conditions required for the desired rearrangement.

The rearrangement reforms the hydroxyl groups originally consumed in formation of the ether and leaves the vinyl benzyl moieties directly bonded to the aromatic ring of the resulting phenol at the ortho position relative to the reformed hydroxyl group. The result is a vinyl functionalized photoactive material. Where the starting photoactive material was a phenol red or phenolphthalein derivative as discussed above, the vinyl functionalized photoactive material will be in accordance with the structural formula:

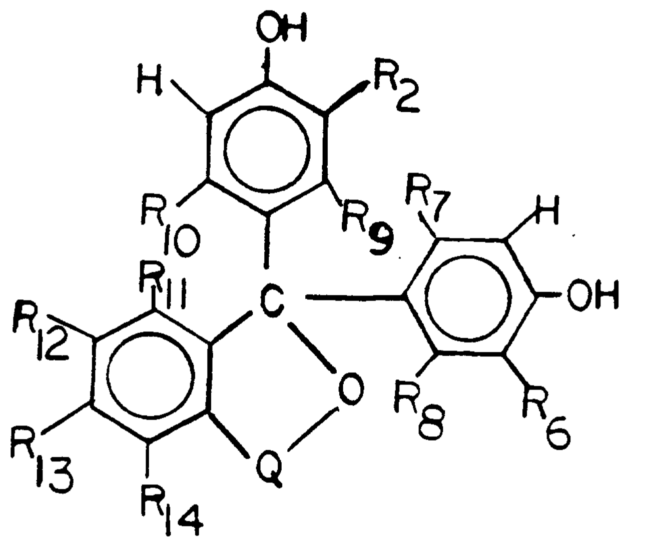

III

in which R2 is the vinylbenzyl moiety discussed above viz.:

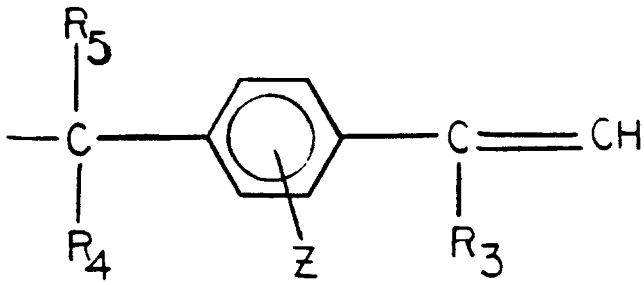

II

Each of R7-R14 are as discussed above. R6 may be R15 discussed above or else may be a vinyl benzyl group conforming to the same structural formula as R2. Formation of the di-functional material, with

both R2 and R6 being vinyl benzyl groups, is favored by employing an excess of vinyl benzyl halide to photoactive material greater than about 2:1. Ordinarily, R2 and R6 are identical, inasmuch as both derive from the vinyl benzyl halide reagent. However, where plural vinyl benzyl halide reagents with differing substituents are employed in the reaction mixture, R2 and R6 may incorporate differing substituents.

The vinyl functionalized photoactive material can be isolated readily from the reaction mixture. Typically, the vinyl functionalized material is insoluble in water and can be precipitated from the reaction mixture by cooling the reaction mixture to about 20°C or below and by adding water to the reaction mixture. The resulting precipitate can be recovered by mechanical separation such as filtration and may be purified by washing with water.

The vinyl functionalized photoactive material can be polymerised by itself or, preferably, with one or more additional vinyl monomers to form a photoactive polymer. Substantially any vinyl monomer can be employed as the comonomer. Preferred comonomers include styrene and derivatives thereof, and acrylic monomers such as methyl and ethyl acrylate and methacrylate and derivatives of such acrylic monomers. Among the derivatives of acrylic monomers which can be employed are the ionomeric derivatives, incorporating an ionic moiety such as ammonium or other cationic moiety bound to an anionic moiety such as a methosulfate. The choice of comonomer ordinarily will not affect the properties of the photoactive moiety directly. However, the choice comonomer will influence the permeability of the resulting polymer. For example, incorporation of acrylic monomers, and particularly the ionomeric monomers will enhance permeability of the polymer to $H+$ ions. These comonomers accordingly are preferred for incorporation in polymers containing a pH sensitive dye moiety to be used as a pH or $CO_2$ sensitive element. More than one vinyl functionalized photoactive material may be incorporated in the polymer. Merely by way of example, a vinyl functionalized light absorbing dye according to the present invention may be copolymerized with a vinyl functionalized luminescent material.

Polymerization can be conducted using conventional polymerization initiation schemes. Among the suitable polymerization initiators are the so called free-radical initiators which undergo conditions such as mildly elevated temperature or exposure to light. Peroxide and azo initiators which undergo thermal decomposition to provide free radicals are particularly preferred. Azo-bis-isobutyronitrile is a particularly preferred initiator. The polymerization reaction typically is performed in solution, in a solvent such as a lower alcohol, preferably ethanol or isopropanol containing all of the desired monomers. The polymerization reaction may take place under conventional conditions such as mildly elevated temperatures, typically about 40°C- 80°C and desirably about 65°C, typically a period of about 24 hours or more. The resulting polymer may be purified by conventional techniques such as ultrafiltration and processed into the desired physical form by conventional polymer processing techniques. In particular, the polymers may be dissolved and solution cast or coated on substrates. In particular, a mass of photoactive polymer may be applied on the tip of a fiber optic by dipping the tip of the fiber optic into a solution of the photoactive polymer, withdrawing the tip and drying the resulting droplet, then repeating the process as needed to accumulate the desired mass. Solutions of the photoactive polymer may contain other dispersed or dissolved photoactive materials. For example, a luminescent material or a polymer incorporating a luminescent material may be dispersed or dissolved in a solution containing a polymer having light absorbing dye moieties.

Thus, the polymer has first repeating units according to the formula:

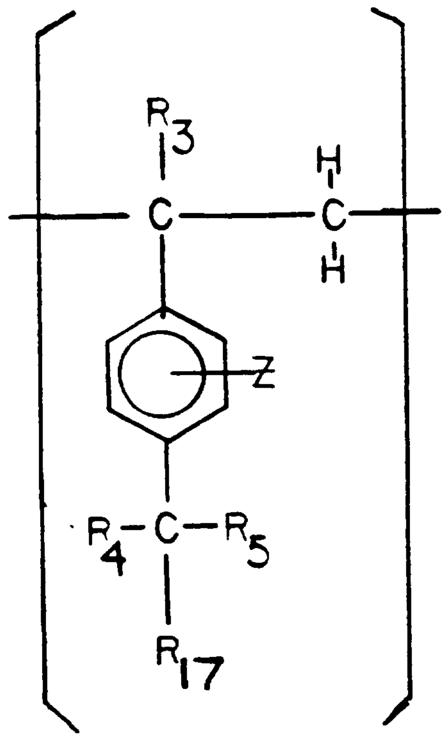

VIII

where R3-R5 are as defined above, and R17 is a photoactive moiety. The polymer also includes one or more additional repeating units according to the formula:

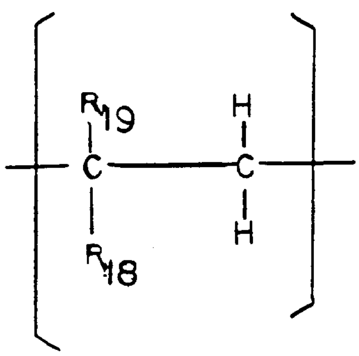

IX

in which, in each additional repeating unit, R18 and R19 are independently selected from the group consisting of H and organic moieties. Preferably, each of R18 and R19 is independently selected from the group consisting of H, alkyl, substituted and unsubstituted benzyl and esters, i.e., $C00R_{20}$ in which $R_{20}$ is an organic moiety. Where the polymer is formed from ethyl and methyl acrylate and methacrylate monomers, $R_{18}$ may incorporate -C00CH3 or $-C00CH_2CH_3$ and $R_{19}$ may be selected from the group consisting of -CH3 or H.

Where the vinyl functionalized photoactive moiety is difunctional, having two phenol groups and two vinylbenzyl moieties bound to these two functional groups, the first repeating units may be in accordance with the formula:

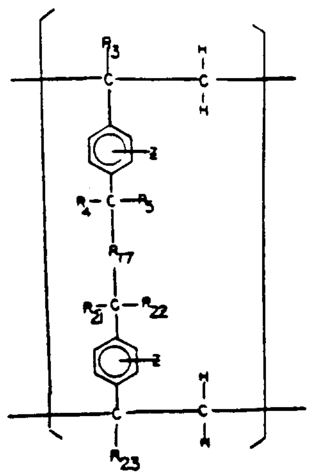

X

wherein each of the vinyl benzyl moieties is bound to a different phenol moiety of R17, $R_{21}$, $R_{22}$ and $R_{23}$ and Z being selected from the same groups as discussed above with reference to $R_3$, $R_4$ and $R_5$. In a polymer incorporating repeating units of this type, the $CR_{23}$-CH2 moiety and the $CR_3$-$CH_2$ moiety lie in different chains of the polymer, so that the polymer chains are cross-linked through the photoactive moiety.

The following non-limiting examples illustrate certain features of the invention:

## EXAMPLE I - SYNTHESIS OF A VINYL FUNCTIONALIZED DYE

Dimethylformamide (DMF) is dried over molecular sieves. 0.16g NaOH is added to 30 ml of the dried DMF. 3.544g of phenol red is then dissolved in this mixture. 4.575g para vinyl benzyl chloride is added while stirring to form a reaction mixture. The reaction mixture is stirred at room temperature for about 2 hours and then held at reflux temperature (approximately 154 ° C) for 48 hours. The reaction mixture is then cooled to room temperature. Upon addition of 250 ml of water, a precipitate forms. The precipitate is washed 5 times with 1000 ml $H_2O$, recovered by filtration and dried at room temperature to yield 3.8g of a phenol red derivative having two vinylbenzyl functionalities, hereinafter referred to as "2-VB-phenol red."

## EXAMPLE II - PREPARATION OF A PHOTOACTIVE POLYMER

40 mg of azo-bis isobutyronitrile ("AIBN") is dissolved in 4 ml ethanol. 0.35G of 2-VB-Phenol Red (from Example I) is added along with 3.2G methylmethacrylate and 5.03G of a 40% aqueous solution of trimethylammonium methylmethacrylate methosulfate ("TMAS"). After polymerization at 65 ° C for 28 hours, a terpolymer of 2-VB-phenol red, methylmethacrylate and TMAS is recovered by evaporating the solvent. The recovered polymer is dissolved in 2-methoxyethanol and purified by filtration using a filter having a 100,000 nominal molecular weight cut-off. Polymer films are formed on substrates by coating the purified solution of the polymer in 2-methoxyethanol on the substrates and drying at room temperature overnight. The resulting films are hydrophilic. The hydrated film has an absorption peak at about 570 nm. The absorptivity at this peak varies strongly with pH over the range 6.8-7.8.

## EXAMPLE III - PHENOLPHTHALEIN

Example I is repeated except that phenolphthalein is employed instead of phenol red. The product is the difunctional vinylbenzyl derivative of phenolphthalein ("2-VB-PP"). 2-VB-PP is copolymerized with methylmethacrylate and TMAS, and the resulting polymer is purified and cast into a film as described in Example II. The resulting film, when hydrated, has an absorption band in the red region. As the film is immersed in solutions of progressively higher pH, the absorption in this band increases substantially.

## EXAMPLE IV - PREPARATION OF A pH-SENSITIVE ELEMENT

4.3 mg of oxazine perchlorate is dissolved in 10 ml of the solution incorporating 2-VB-phenol red in 2-methoxyethanol prepared in Example II. The resulting solution is dried to form a film of the phenol red bearing co-polymer having oxazine perchlorate dispersed therein. The resulting polymer film is hydrated

and exposed to environments of varying pH. The film emits response light at about 640-750 NM upon illumination with excitation light at 560 NM or at 620 NM. As the pH of the environment increases, the magnitude of the response light evoked by 560 NM excitation light decreases substantially, whereas the magnitude of the response light evoked by 620 NM excitation light does not change appreciably.

EXAMPLE V - PREPARATION OF A $CO_2$-SENSITIVE ELEMENT

A solution is prepared as in Example IV, except that the solution contains 20 milimolar $NaHC0_3$. A film is formed from this solution, coated with silicone and hydrated. The coated, hydrated film is then exposed to gas mixtures containing varying amounts of $CO_2$. As the $CO_2$ concentration in the environment increases, the magnitude of response light evoked by excitation light at 560 NM increases over the range of $CO_2$ concentration 0-10%, whereas the magnitude of response light evoked by 620 NM excitation light is substantially constant.

As numerous variations and combinations of the features described above can be utilized without departing from the present invention, the foregoing description of the preferred embodiments should be taken by way of illustration rather than by way of limitation of the present invention as defined in the claims.

## Claims

**1.** Apparatus for monitoring a condition comprising:

(a) a support;

(b) a luminescent material substantially insensitive to the condition to be monitored, said luminescent material being adapted to emit light within a response band of wavelengths in response to measurement excitation light within a measurement band of excitation wavelengths and in response to reference excitation light within a reference band of excitation wavelengths different than said measurement band,said luminescent material being mounted on said support;

(c) a light-absorbing material sensitive to said condition mounted on said support so that said light-absorbing material can be exposed to the condition to be sensed, said light-absorbing material having absorptivity for light in said measurement band of excitation wavelengths varying in response to the condition to be sensed;

(d) illumination means for providing measurement and reference excitation light so that the ratio of the intensity of light in said measurement band to the intensity of light in said reference band varies with time and directing such excitation light to said luminescent material so that the transmitted measurement excitation light passes through said light-absorbing material;and

(e) sensor means for monitoring response light emitted by said luminescent material.

**2.** Apparatus as claimed in claim 1 wherein said sensor means includes signal processing means for providing a measurement signal indicating the magnitude of response light produced responsive to said measurement excitation light and a reference signal indicating the magnitude of response light produced responsive to said reference excitation light.

**3.** Apparatus as claimed in claim 2 wherein said illumination means includes means for providing measurement excitation light and reference excitation light alternately, said sensor means includes means for providing a response signal representing the magnitude of response light transmitted from said luminescent material, said signal processing means including means for sampling said response signal in synchronism with said alternating light.

**4.** Apparatus as claimed in claim 2 further comprising means for comparing said measurement signal and said reference signal and deriving an output signal representative of the condition to be monitored based upon such comparison.

**5.** Apparatus as claimed in claim 4 wherein said means for comparing includes means for dividing the magnitude represented by said measurement signal by the magnitude represented by said reference signal to thereby derive a ratio between the magnitude of response light produced responsive to measurement excitation light and the magnitude of light produced responsive to reference excitation light.

**6.** Apparatus as claimed in claim 1 wherein said luminescent material and said light absorbing material are mounted to said support so that at least a part of said excitation light transmitted to said luminescent material passes through said light absorbing material, and wherein said light absorbing material has substantially no variation in absorptivity for light in said reference excitation wavelength band with the condition to be monitored.

**7.** Apparatus as claimed in claim 6 wherein said luminescent material and said light-absorbing material are mounted to said support so that at least part of the response light passing from said luminescent material to said sensor means passes through said light absorbing material, and wherein said light absorbing material has substantially no variation in absorptivity for light in said response wavelength band with the condition to be monitored.

**8.** Apparatus as claimed in claim 1 wherein said support includes an elongated light-transmitting probe having proximal and distal ends, said luminescent material and said light-absorbing material being mounted to said distal end of said probe, said illumination means being arranged to transmit said measurement and reference excitation light from said proximal end of said probe to said luminescent material via said probe, said sensor means being arranged to monitor response light transmitted to the proximal end of said probe.

**9.** Apparatus as claimed in claim 8 wherein said probe has a single optically transmissive fiber extending from proximal end to said distal end.

**10.** Apparatus as claimed in claim 8 wherein said light-absorbing material and said luminescent material are disposed in a single mass at the distal end of said probe.

**11.** Apparatus as claimed in claim 8 wherein said light absorbing material is positioned between the proximal end of said probe and said luminescent material.

**12.** Apparatus as claimed in claim 1 wherein said light absorbing material has substantially no absorptivity for light in said reference excitation wavelength band.

**13.** Apparatus as claimed in claim 1 wherein said light-absorbing material includes a pH-sensitive dye.

**14.** Apparatus as claimed in claim 13 further comprising a $CO_2$ sensitive buffer and a $CO_2$ permeable matrix, said buffer and said pH-sensitive dye being disposed in said matrix.

**15.** Apparatus as claimed in claim 1 wherein said measurement band includes wavelengths of about 520 to about 580 nm, said reference band includes wavelengths of about 590 to about 640 nm and said response band includes wavelengths of about 610 to about 710 nm.

**16.** Apparatus as claimed in claim 15 wherein said light-absorbing material includes a phenol red moiety.

**17.** Apparatus as claimed in claim 1 wherein said luminescent material includes a material selected from the group consisting of oxazine 1 perchlorate oxazine 4 perchlorate, oxazine 170 perchlorate, Nile Blue A Perchlorate, Cresyl Violet Perchorate, Sulfomodamine 101, Sulforhodamine B, rhodamine, and porphyrin.

**18.** A vinyl-functionalized photoactive material according to the formula:

$$R_1[R_2]_{n_1} \qquad \text{I}$$

wherein:

$n_1$ is an integer greater than or equal to 1;

$R_1$ is a photoactive organic moiety having one or more phenol moieties,the number of said phenol

moieties being at least equal to $n_1$;

each $R_2$ is independently selected from the group consisting of moieties according to the formula:

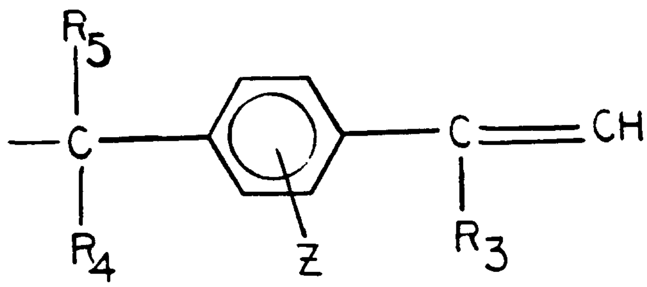

II

wherein:

each of $R_3$,$R_4$ and $R_5$ is independently selected from the group consisting of H aryl and lower alkyl; and

Z represents moieties bound to the aromatic ring, selected from the group consisting of H, aryl and lower alkyl,

each said $R_2$ being bound to a separate one of said phenol moieties of $R_1$ at the ortho position with respect to the -OH group of such phenol moiety.

**19.** A material as claimed in claim 18 wherein $R_1$ is a pH-sensitive organic dye moiety.

**20.** A vinyl-functionalized dye according to the formula:

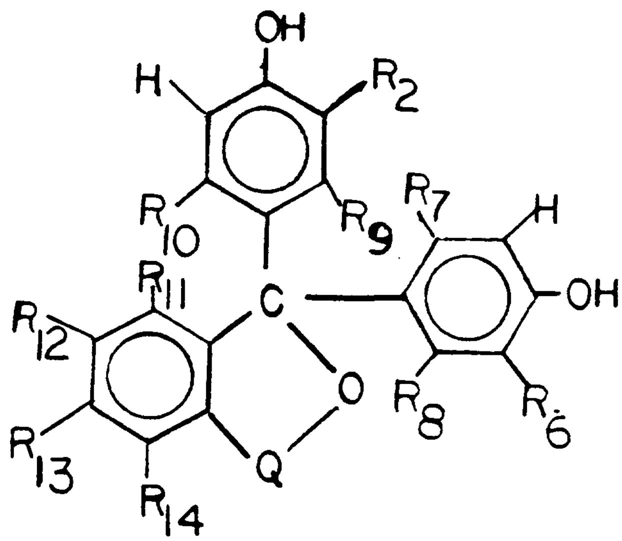

III

wherein:

Q is a moiety selected from the group consisting of:

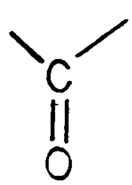

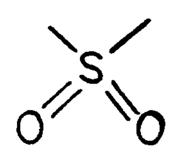

IV & V

$R_2$ is a moiety according to the formula:

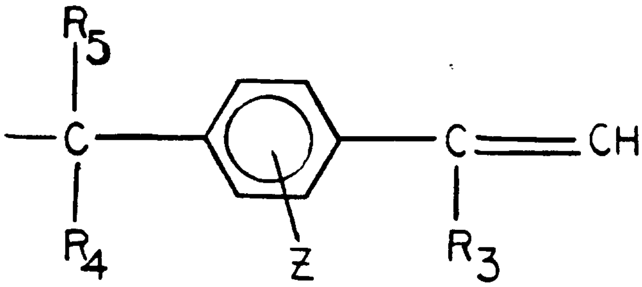

II

each of $R_3$,$R_4$ and $R_5$ is independently selected from the group consisting of H, aryl and lower alkyl;

Z represents moieties bound to the aromatic ring, selected from the group consisting of H, aryl and lower alkyl;

$R_6$ is selected from the group consisting of $R_2$, lower alkyls, H and halogens;and

each of $R_7$ through $R_{14}$ is independently selected from the group consisting of lower alkyl,H and halogens.

**21.** A dye as claimed in claim 20 wherein $R_6$ is $R_2$.

**22.** A dye as claimed in claim 20 wherein $R_6$ is selected from the group consisting of lower alkyl, H and halogens.

**23.** A dye as claimed in claim 20 wherein Q is:

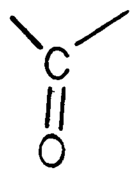

IV

**24.** A dye as claimed in claim 23 wherein each of $R_7$ through $R_{14}$ is H.

**25.** A dye as claimed in claim 20 wherein Q is:

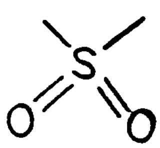

V

**26.** A dye as claimed in claim 25 wherein each of $R_7$ through $R_{14}$ is H.

**27.** A dye as claimed in claim 20 wherein each of $R_3$, $R_4$ and $R_5$ is H and all said Z moieties are H.

**28.** A method of making a vinyl-functionalized photoactive material as claimed in claim 18 comprising the step of reacting a photoactive material having one or more phenol moieties with a vinylbenzylhalide reagent according to the formula X-$R_2$ wherein X is selected from the group consisting of F, C1 and Br, and $R_2$ is a vinylbenzylhalide moiety according to the formula:

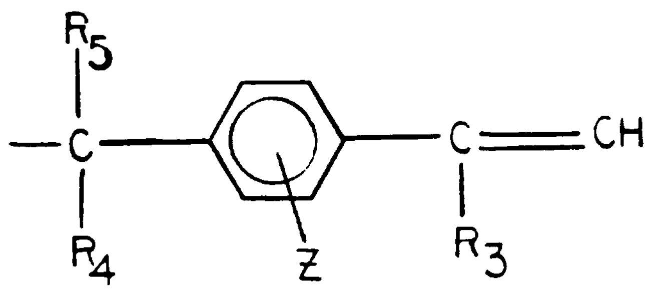

II

wherein:

each of $R_3$,$R_4$ and $R_5$ is independently selected from the group consisting of aryl, lower alkyl and H; and

Z represents moieties bound to the aromatic ring, selected from the group consisting of H, aryl and lower alkyl.

**29.** A method as claimed in claim 28 wherein said reacting step includes the steps of contacting said photoactive material with said vinylbenzylhalide reagent so that $R_2$ replaces H at the hydroxyl group of said one or more phenol moieties of said photoactive material to form an intermediate product, and reacting said intermediate product so as to reform the hydroxyl group and reposition $R_2$ to the other position with respect to said reformed hydroxyl group.

**30.** A method as claimed in claim 28 wherein said reacting step includes the steps of mixing said photoactive material with said vinylbenzylhalide reagent and a solvent to form a reaction mixture maintaining said reaction mixture at an elevated temperature and then isolating said vinyl-functionalized photoactive material from said reaction mixture.

**31.** A method as claimed in claim 28 wherein said photoactive material is a pH-sensitive dye according to the formula:

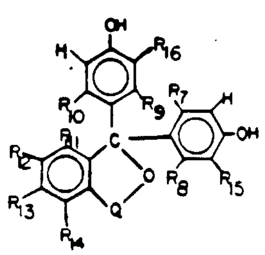

VII

wherein:

each of $R_7$ through $R_{16}$ is independently selected from the group consisting of lower alkyl,H and halogens.

**32.** A method as claimed in claim 31 wherein at least one of $R_{15}$ and $R_{16}$ is H.

**33.** A method as claimed in claim 31 wherein both of $R_{15}$ and $R_{16}$ are H and wherein said step of reacting said dye with said vinylbenzylhalide reagent is performed at a starting molar ratio of said reagent to said dye of at least about 2:1.

**34.** A method of making a polymeric pH-sensitive light-absorbing material comprising the steps of copolymerizing a vinyl-functionalized photoactive material as claimed in claim 18 with one or more vinyl monomers other than said photoactive material.

**35.** A photoactive polymer having first repeating units according to the formula: wherein:

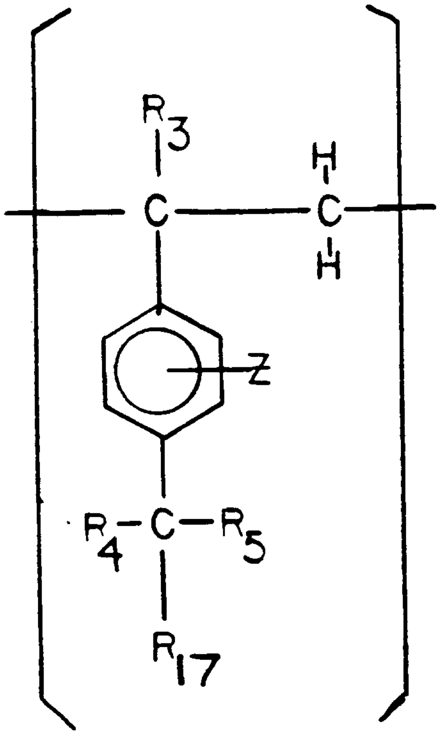

VIII

$R_{17}$ is a photoactive moiety having one or more phenol moieties, said $R_4$-C-$R_5$ moiety being bound to one of said phenol moieties at the ortho position with respect to the -OH group of such phenol moiety;

each of $R_3$,$R_4$ and $R_5$ is independently selected from the group consisting of aryl, lower alkyl and H; and

Z represents moieties bound to the aromatic ring, selected from the group consisting of H, aryl and lower alkyl.

**36.** A polymer as claimed in claim 35 wherein $R_{17}$ is a pH-sensitive organic dye moiety.

**37.** A polymer as claimed in claim 35, further comprising uniform or nonuniform additional repeating units according to the formula:

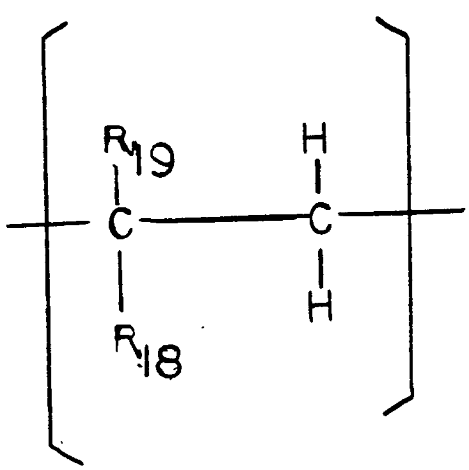

IX

wherein in each said additional repeating unit:
$R_{18}$ and $R_{19}$ are each independently selected from the group consisting of H and organic moieties.

**38.** A polymer as claimed in claim 37, wherein $R_{18}$ and $R_{19}$ are each independently selected from the group consisting of H, alkyl, substituted and unsubstituted benzyl, and $-COOR_{20}$, wherein $R_{20}$ is an organic moiety.

**39.** A polymer as claimed in claim 38 wherein, in at least some of said additional repeating units, $R_{14}$ is $-COOCH_3$ and $R_{19}$ is selected from the group consisting of $-CH_3$ and -H.

**40.** A polymer as claimed in claim 38 wherein, in at least some of said additional repeating units, $R_{18}$ includes an ionic moiety.

**41.** A polymer as claimed in claim 40 wherein, in at least some of said additional repeating units,$R_{18}$ includes an ammonium moiety.

**42.** A polymer as claimed in claim 37 wherein at least some of said additional repeating units are trimethylammonium methylmethacrylate methosulfate.

**43.** A polymer according to claim 35 wherein $R_{17}$ has two phenol moieties and said first repeating units are according to the formula:

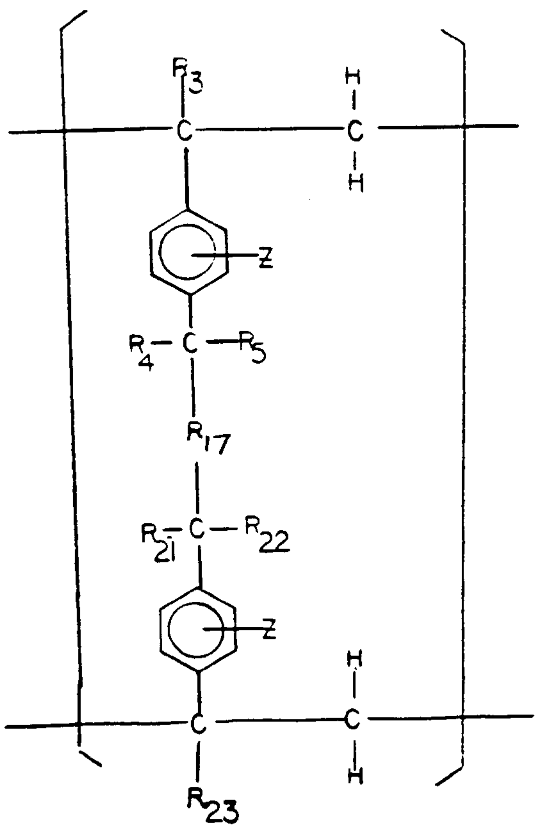

X

wherein:

said $R_{21}$-C-$R_{22}$ moiety being bound to one of said phenol moieties at the ortho position with respect to the -OH group of such phenol moiety; and

each of $R_{21}$, $R_{22}$ and $R_{23}$ is independently selected from the group consisting of lower alkyls and H.

**44.** A polymer as claimed in claim 43 further comprising uniform or nonuniform additional repeating units, said repeating units defining generally linear vinyl polymer chains, the $-CR_3-CH_2-$ moiety and the $-CR_{23}-CH_2-$ moiety of each said first repeating unit being polymerized in different ones of said chains, whereby said chains are cross-linked through said $R_{17}$ moieties.

**45.** A condition-sensitive element comprising:
(a) a support;
(b) a luminescent material substantially insensitive to the condition to be monitored, said luminescent material being adapted to emit light within a response band of wavelengths in response to measurement excitation light within a measurement band of excitation wavelengths and in response to reference excitation light within a reference band of excitation wavelengths different than said measurement band, said luminescent material being mounted on said support;
(c) a light-absorbing material sensitive to said condition mounted on said support so that said light-absorbing material can be exposed to the condition to be sensed, said light-absorbing material having absorptivity for light in said measurement band of excitation wavelengths varying in response to the condition to be sensed.

**46.** An element as claimed in claim 45 wherein said support includes an elongated light-transmitting probe having proximal and distal ends, said luminescent material and said light-absorbing material being mounted adjacent said distal end of said probe.

**47.** An element as claimed in claim 46 wherein said probe has a single optically transmissive fiber extending from proximal end to said distal end.

**48.** An element as claimed in claim 47 wherein said light-absorbing material and said luminescent material are disposed in a single mass at the distal end of said probe.

**49.** An element as claimed in claim 46 wherein said light-absorbing material includes a pH-sensitive dye.

**50.** An element as claimed in claim 49 further comprising a $CO_2$ sensitive buffer and a $CO_2$ permeable matrix, said buffer and said pH-sensitive dye being disposed in said matrix.

**51.** Apparatus as claimed in claim 49 wherein said light-absorbing material includes a dye moiety selected from the group consisting of phenol red and phenolphthalein.

**52.** Apparatus as claimed in claim 51 wherein said luminescent material includes a material selected from the group consisting of oxazine 1 perchlorate, oxazine 4 perchlorate, oxazine 170 perchlorate, Nile Blue A Perchlorate, Cresyl Violet Perchlorate, Sulforhodamine 101, Sulforhodamine B, rhodamine and porphyrin.

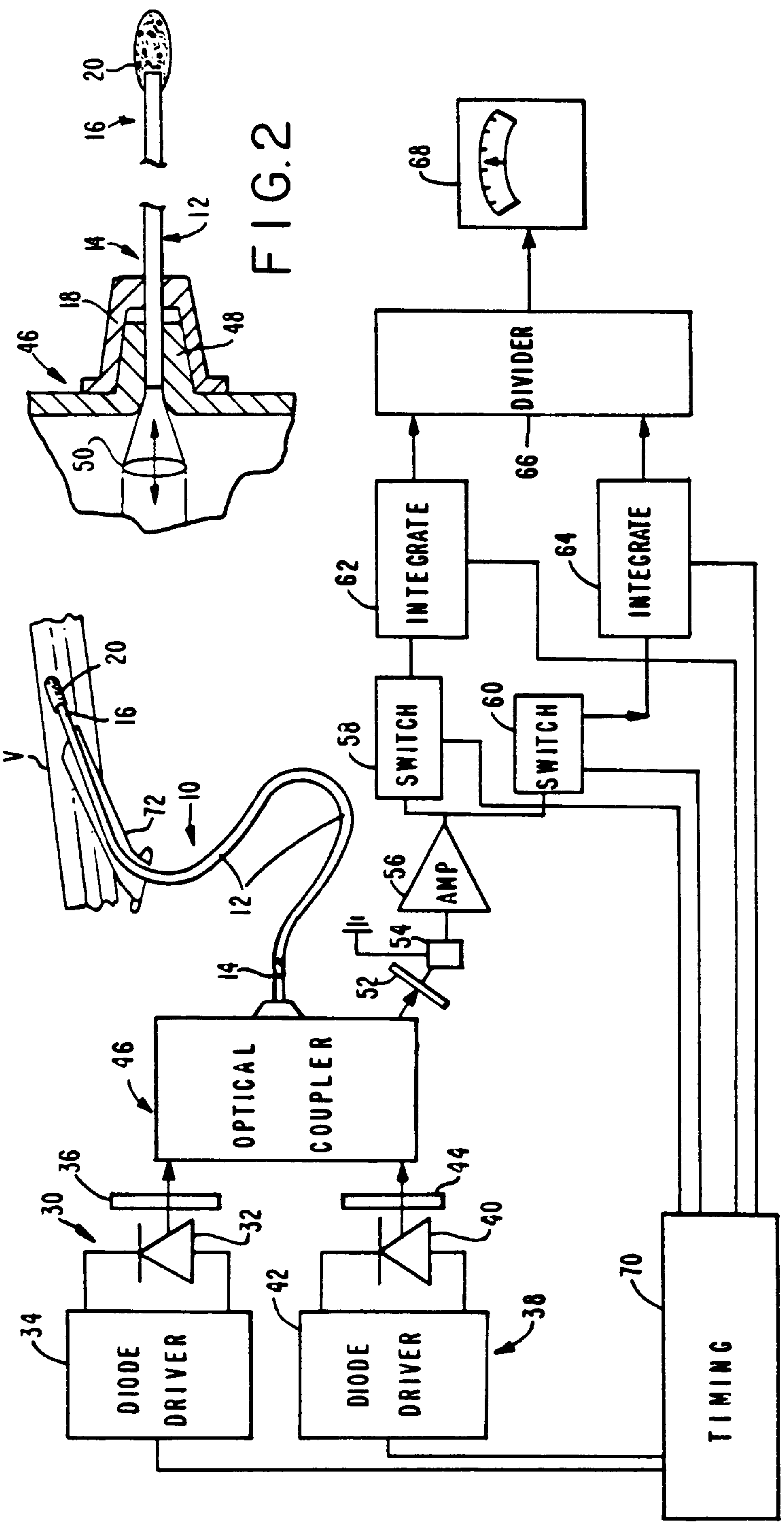
DIODE DRIVER
DIODE DRIVER
OPTICAL COUPLER
AMP
SWITCH
SWITCH
INTEGRATE
INTEGRATE
DIVIDER
TIMING

FIG. 2

FIG. 1

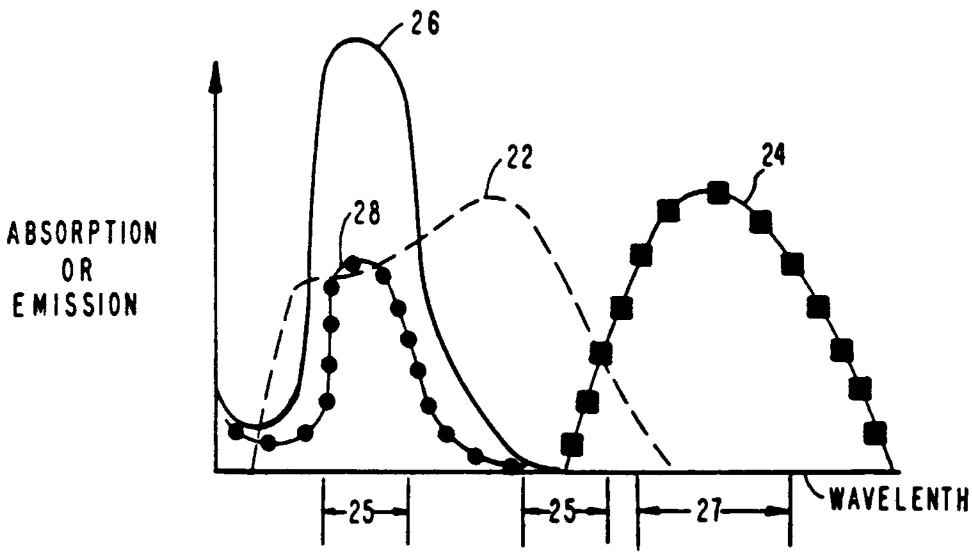


FIG. 3

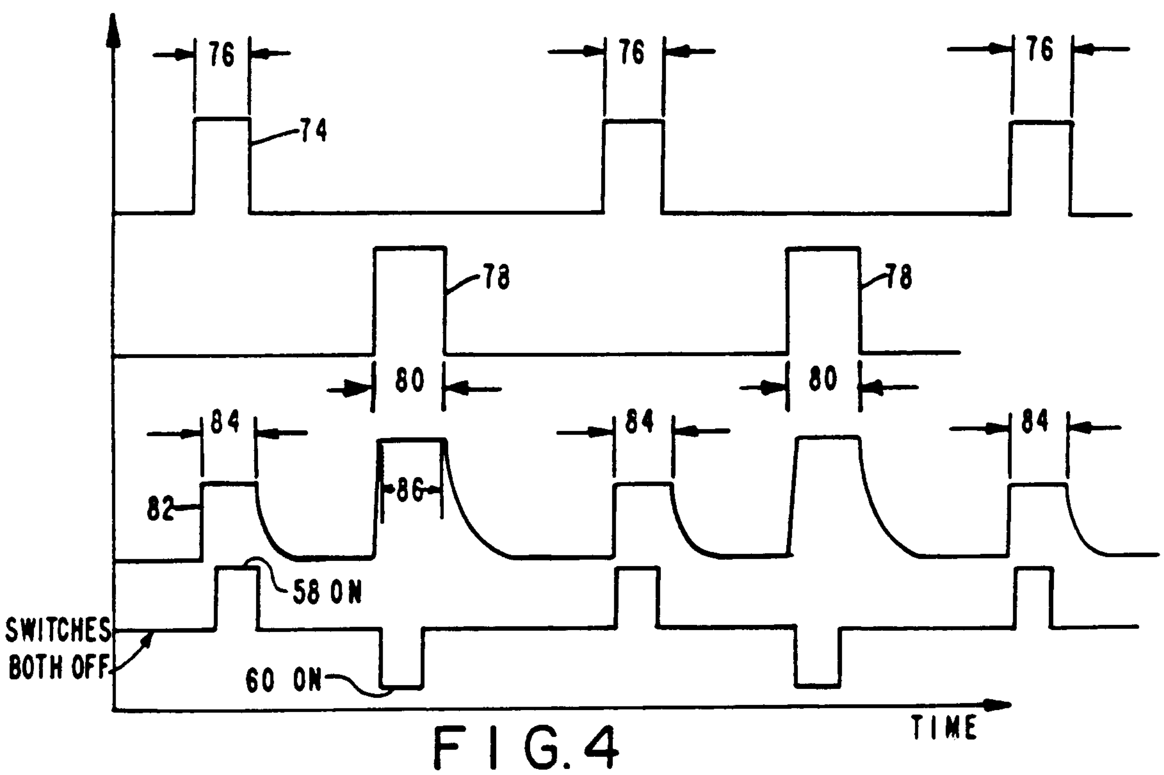


FIG.4

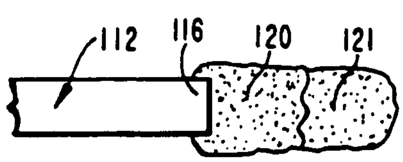


FIG. 5